# EUROPÄISCHE PATENTSCHRIFT

(11) **EP 1 279 657 B1**
(45) Veröffentlichungstag und Bekanntmachung des Hinweises auf die Patenterteilung: **29.03.2006**
(21) Anmeldenummer: 02015586.7
(22) Anmeldetag: 15.07.2002
(51) Int. Cl.: C07B 41/06, C07C 45/37, C07D 333/22, C07D 307/46

(54) **Verfahren zur Herstellung von Carbonylverbindungen aus Alkoholen**
Process for preparing carbonyl compounds from alcohols
Procédé pour la préparation de composés carbonyliques à partir d'alcools

(30) Priorität: 26.07.2001 DE 10137455
(43) Veröffentlichungstag der Anmeldung: 29.01.2003
(73) Patentinhaber: LANXESS Deutschland GmbH, 51369 Leverkusen (DE)
(72) Erfinder: Eckert, Markus, Dr., 50937 Köln (DE); Militzer, Hans-Christian, Dr., 51519 Odenthal (DE); Beller, Matthias, Prof. Dr., 18211 Ostseebad Nienhagen (DE); Döbler, Christian, Dr., 18107 Lichtenhagen-Dorf (DE); Mehltretter, Gerhard, 80993 München (DE); Sundermeier, Uta, 18055 Rostock (DE)

(56) Entgegenhaltungen:
- US-A- 4 306 083
- SOMAIAH P V ET AL: "Kinetics of oxidation of alcohols, diols and alpha-hydroxy acids by osmium(VIII) in aqueous alkaline medium: evidence in support of hydride ion abstraction mechanism" INDIAN JOURNAL OF CHEMISTRY, SECTION A, Bd. 27A, Nr. 10, 1988, Seiten 876-879, XP008012564
- CHEMICAL ABSTRACTS, vol. 108, no. 17, 25. April 1988 (1988-04-25) Columbus, Ohio, US; abstract no. 149742z, SOMAIAH P V ET AL: "Role of osmium tetroxide in oxidation of 2-propanol in presence and absence of different one- and two-equivalent oxidants in aqueous alkaline medium" Seite 674; XP002227272 & INDIAN J. CHEM., SECT. A, Bd. 26A, Nr. 5, 1987, Seiten 402-406,
- CHEMICAL ABSTRACTS, vol. 99, no. 25, 19. Dezember 1983 (1983-12-19) Columbus, Ohio, US; abstract no. 211922j, NAIDU H M K ET AL: "Kinetics and mechanism of osmium(VIII) and ruthenium(III) catalyzed oxidation of ethanediol and 1,2-propanediol with chloramine-T in alkaline and acid media" Seite 592; XP002227273 & Z. PHYS. CHEM. (LEIPZIG), Bd. 264, Nr. 3, 1983, Seiten 469-480,
- MAIONE A M ET AL: "Carbonyl compounds by osmium tetroxide oxidation: preferential oxidation of primary over secondary hydroxy groups" SYNTHESIS, Nr. 11, November 1984 (1984-11), Seiten 955-957, XP002175379
- COLEMAN K S ET AL: "Catalytic oxidation of alcohols into aldehydes and ketones by an osmium-copper bifunctional system using molecular oxygen" TETRAHEDRON LETTERS, Bd. 40, Nr. 19, 7. Mai 1999 (1999-05-07), Seiten 3723-3726, XP004163815

## Beschreibung

Die vorliegende Erfindung betrifft ein neues Verfahren zur Herstellung von Carbonylverbindungen durch Oxidation von Alkoholen in Gegenwart von Katalysatoren auf Basis von Osmiumverbindungen.

Als bedeutende Carbonylverbindungen sind Aldehyde und Ketone anzusehen, die als chemische Zwischenprodukte insbesondere als Feinchemikalien sowie als Vorprodukte für Farbstoffe, Arzneimittel und Agrowirkstoffe Verwendung finden.

Obwohl aus der Literatur bereits eine große Zahl von Herstellverfahren für Aldehyde und Ketone bekannt sind, besteht aufgrund der Vielzahl unterschiedlicher, technisch interessanter Carbonylverbindungen großes Interesse an neuen umweltfreundlichen katalytischen Synthesemethoden. Industrielle Anwendung zur Aldehydsynthese finden beispielsweise die Hydroformylierung von Olefinen, die Formylierung von Aromaten, die Ozonolyse von Olefinen und die Oxidation von Alkoholen. Von den genannten Syntheseverfahren sind für Feinchemikaliensynthesen insbesondere die Oxidation von Alkoholen interessant. Aufgrund der Verfügbarkeit und des Preises sind Alkohole ideale Ausgangsmaterialien für Aldehyd- und Ketonsynthesen. Oxidationen von Alkoholen zu Carbonylverbindungen gelingen in Gegenwart von stöchiometrischen Mengen an Chromverbindungen, mit Dimethylsulfoxid/Oxalylchlorid, Periodat, Braunstein oder anderen Reagentien. Aus ökologischer Sicht - aber auch ökonomisch - sind Verfahren mit kostengünstigen Oxidationsmitteln wie Wasserstoffperoxid und Bleichlauge in Gegenwart von Katalysatoren interessanter. Vorteilhafter sind jedoch katalytische Verfahren, die mit Sauerstoff als Oxidationsmittel gelingen. Aus der Literatur bekannt sind metallkatalysierte Oxidationen von Alkoholen mittels Sauerstoff, wobei in den meisten Fällen Ruthenium- und Palladiumkatalysatoren zum Einsatz kommen. In der Regel werden nur Alkohole mit allylischer oder benzylischer Struktur mit guten Ausbeuten umgesetzt, wobei jedoch von Nachteil ist, dass relativ hohe Katalysatorkonzentrationen notwendig sind, lange Reaktionszeiten erforderlich sind oder Cokatalysatoren zugesetzt werden müssen.

Bei den vorbekannten Verfahren sind die eingesetzten Katalysatormengen für eine industrielle Anwendung zu groß. Die Menge des eingesetzten Edelmetallkatalysators dominiert bei den Reaktionen die Rohstoffkosten und macht die entsprechenden Verfahren unwirtschaftlich. Weiterhin ist es schwierig bei Verwendung von > 1mol% Katalysator, Edelmetallspuren vom Produkt zu trennen. Dies ist jedoch eine Voraussetzung für die Anwendung der Verfahren im Bereich der Herstellung von Pharma- und Agrozwischenprodukten.

Osmium-katalysierte Alkoholoxidationen wurden bisher kaum untersucht.

US-A 4 306 083 beschreibt ein Verfahren zur Oxidation von arylsubstituierten Alkoholen zu den entsprechenden Aldehyden unter Verwendung von molekularem Sauerstoff als Oxidationsmittel und einem Edelmetallkatalysator, der auch Osmium enthalten kann. Indian Journal of Chemistry 1988 (Vol 27A, 876-879) beschreibt die Oxidation von Alkoholen, Diolen und α-Hydroxycarbonsäuren unter Verwendung von OsO₄ in wässrigem alkalischem Medium, wobei jedoch stöchiometrische Mengen an Os-Katalysator benötigt werden. Synthesis 1984, 11, 955-957 beschreibt ein Verfahren zur Oxidation von Cholesterol-Derivaten mit OsO₄ als Oxidationsmittel, wobei hier ein Verzicht auf die Verwendung stöchiometrischer Mengen an OsO₄ zu drastischen Ausbeuteverlusten führt. Chemical Abstracts 1988, Vol. 108, No. 17,149 742, beschreibt em Verfahren zur Oxidation von Alkoholen, wobei OsO₄ sowie als weiteres Reoxidationsmittel Diperiodatocuprat(III) (DCP), Hexacyanoferrat(III) (HCF), Periodat (IO₄) oder Chloramin T (CAT) verwendet werden. Chemical Abstracts 1983, Vol. 99, No. 25,211 922 J beschreibt ebenfalls ein Verfahren zur Oxidation von Alkoholen, wobei OsO₄ und Chloramin T (CAT) verwendet werden.

Beschrieben ist zudem die Oxidation von vorwiegend allylischen und benzylischen primären Alkoholen mit dem System OsO₄ (1mol%) / CuCl (1.5mol%) in Toluol bei 100°C mit Sauerstoff unter Zusatz von Molsieb, es werden bei hoher Selektivität Ausbeuten zwischen 19 und 96% erreicht (vgl. K.S. Coleman, M. Coppe, C.Thomas, J.A. Osbom, *Tetrahedron Lett.* **1999,** *40,* 3723). Auch die Oxidation von 4-Methoxybenzalkohol zu 4-Methoxybenzaldehyd mit Sauerstoff und 1Mol -% OsO₄ in Toluol bei 100°C in Gegenwart von Molsieb ist dort beschrieben. Allerdings wird darauf hingewiesen, dass das Weglassen von CuCl zu einer Abnahme der Katalysatoraktivität führt.

Es bestand daher Bedarf nach einem neuen, technisch durchführbaren Verfahren zur Herstellung von Carbonylverbindungen welches kostengünstig ist, mit geringen Mengen an Metallkatalysatoren auskommt und eine höhe Katalysatorproduktivität ermöglicht.

Überraschenderweise wurde ein Verfahren zur Herstellung von Carbonylverbindungen durch Oxidation von Alkoholen in Gegenwart von Osmiumverbindungen gefunden, dass die oben genannten Bedingungen erfüllt und zudem die Carbonylverbindungen in hoher Ausbeute und Reinheit liefert.

Die vorliegende Erfindung betrifft ein Verfahren zur Herstellung von Carbonylverbindungen der Formel (I) 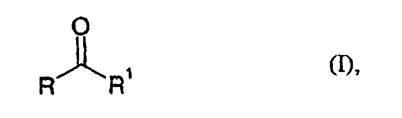 worin
- R und R¹: unabhängig voneinander für Wasserstoff oder für einen jeweils gegebenenfalls substituierten Alkyl-, Cycloalkyl-, Aryl- oder Heteroarylrest stehen,
oder
- R und R¹: gemeinsam mit dem C-Atom an welches sie gebunden sind, für eine Cycloalkylrest stehen,
durch Umsetzung von Alkoholen der allgemeinen Formel (II) 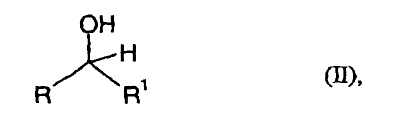 worin R und R¹ die oben genannte Bedeutung besitzen,
mit einem Oxidationsmittel in Gegenwart einer katalytischen Menge eines Osmiumkatalysators in Wasser oder einem Wasser enthaltenden Lösemittelgemisch bei einem pH-Wert von 7 bis 14.

In der Bedeutung von R und R¹ steht Alkyl im allgemeinen für einen geradkettigen oder verzweigten, gegebenenfalls 1- bis 8-fach, gleich oder verschieden substitierten Kohlenwasserstoffrest mit 1 bis 18 C -Atomen. Vorzugsweise steht Alkyl für einen geradkettigen oder verzweigten, gegebenenfalls 1-bis 3-fach gleich oder verschieden substituierten Kohlenwasserstoffrest mit 1 bis 8 C -Atomen. Besonders bevorzugt steht Alkyl für Methyl, Ethyl und Octyl.

In der Bedeutung von R und R¹ steht Cycloalkyl für eine gegebenenfalls 1- bis 8-fach, gleich oder verschieden substituierten cyclischen Kohlenwasserstoffrest mit 5 bis 18 C-Atomen, wobei ein oder mehrere CH₂-Gruppen dieses Cycloalkylrestes durch Heteroatome, bevorzugt N, O oder S ersetzt sein können. Als Cycloalkylreste R oder R¹, bei denen ein oder mehrere CH₂-Gruppen dieses Cycloalkylrestes durch ein oder mehrere Heteroatome, bevorzugt N, O oder S ersetzt sind, sind ein Piperidin- oder Piperazin-Rest geeignet. Vorzugsweise besitzt der Cycloalkylrest 5 bis 10 C-Atome, besonders bevorzugt steht er für Cyclohexyl, wobei wiederum ein oder mehrere CH₂-Gruppen dieses Cycloalkylrestes durch ein oder mehrere Heteroatome, bevorzugt N, O oder S ersetzt sein können.

In der Bedeutung von R und R¹ steht Aryl im allgemeinen für einen unsubstituierten oder 1-bis 8-fach, gleich oder verschieden substituierten, gegebenenfalls annelierten aromatischen Rest mit 6 bis 14 C-Atomen, vorzugsweise 6 bis 10 C-Atomen, insbesondere für gegebenenfalls 1-bis 3-fach, gleich oder verschieden substituiertes Phenyl oder Naphthyl, insbesondere bevorzugt für Phenyl, p-Methylphenyl oder p-Methoxyphenyl.

In der Bedeutung von R und R¹ steht Heteroaryl im allgemeinen für einen unsubstituierten oder 1-bis 8-fach, gleich oder verschieden substituierten, gegebenenfalls annelierten aromatischen Rest mit 4 bis 14 C-Atomen vorzugsweise 5 bis 10 C-Atomen, insbesondere 5 bis 7 C-Atomen und 1 bis 3, vorzugsweise 1 oder 2 Heteroatomen aus der Reihe N, O, S, ganz besonders bevorzugt steht Heteroaryl für Furan und Thiophen.

Die genannten Alkyl-, Cycloalkyl-, Aryl- oder Heteroarylreste sind jeweils gegebenenfalls 1- bis 8-fach, gleich oder verschieden substituiert durch Alkyl, CN, COOH, COO-Alkyl, COO-Aryl, CO-Alkyl, CO-Aryl, O-Alkyl, O-Aryl, O-CO-Aryl, O-CO-Alkyl, OCOO-Alkyl, N-Alkyl₂, NH-Alkyl, N-Aryl₂, NH-Aryl, NO, NO₂, NOH, Aryl, Fluor, Chlor, Brom, Iod, NO₂, SiAlkyl₃, CHO, SO₃H, SO₃-Alkyl, SO₂-Alkyl, SO-Alkyl, CF₃, NHCO-Alkyl, CONH₂, CONH-Alkyl, NHCOH, NHCOO-Alkyl, CHCHCO₂-Alkyl, CHCHCO₂H, PO-(Aryl)₂, PO-(Alkyl)₂, PO₃H₂, PO(O-Alkyl)₂, wobei die genannten Alkyl- und Arylreste wiederum die oben angegebenen allgemeinen und bevorzugten Bedeutungen haben.

Insbesondere werden zur Herstellung von Carbonylverbindungen der Formel (I) Alkohole der Formel (II) eingesetzt, worin mindestens einer der Reste R oder R¹ für eine Aryl- oder Heteroarylgruppe steht, die die oben genannte Bedeutung besitzt.

Die Verbindungen der Formel (II) können einzeln oder im beliebigen Gemisch untereinander eingesetzt werden.

Das erfindungsgemäße Verfahren wird in Gegenwart von Wasser als Lösungsmittel durchgeführt. Dabei bildet sich mit dem Alkohol der Formel (II) in der Regel ein zweiphasiges Lösungsmittelsystem. Grundsätzlich kann neben dem Alkohol der Formel (II) ein weiteres organisches Lösemittel zugesetzt werden. Hierbei werden üblicherweise 1 bis 5 l, bevorzugt 2 bis 31/mol Alkohol der Formel (II) eingesetzt. Als weitere Lösungsmittel werden aliphatische Ether, tertiäre Alkohole und Ester sowie deren Gemische verwendet.

Das erfindungsgemäße Verfahren wird bei einem pH-Wert im Bereich zwischen 7 und 14, vorzugsweise zwischen 9 bis 10, insbesondere zwischen 10 bis 11 durchgeführt. Dabei wird im allgemeine als Wasserphase eine wässrige Lösung mit einem pH-Wert von 7 bis 14 verwendet. Der basische pH-Wert der Lösung wird durch den Zusatz einer Base zum Wasser erzielt. Generell ist es vorteilhaft in gepufferten wässrigen Lösungen, vorzugsweise bei pH 9 bis 13 zu arbeiten. Der basische pH-Wert wird durch den Zusatz von bekannten Puffern zu Wasser oder durch Titration einer Base zum Reaktionsgemisch eingestellt. Mitunter ist es für die Abtrennung der Carbonylverbindungen vorteilhaft, wenn an Stelle von Wasser oder gepufferten wässrigen Lösungen als Lösungsmittel eine wässrige Salzlösung oder gepufferte wässrige Salzlösung - beispielsweise wässrige Lösung eines Alkali- oder Erdalkalihalogenids - eingesetzt wird.

Für die Durchführung des erfindungsgemäßen Verfahrens werden als Oxidationsmittel molekularer Sauerstoff oder eine Gasmischung, die molekularen Sauerstoff enthält, oder Alkali- oder Erdalkali-Hypochlorit-Lösungen verwendet.

Von den Gasmischungen bevorzugt sind solche, die mindestens 15 Volumenprozent Sauerstoff enthalten. Besonders bevorzugt sind Luft und Gasmischungen mit einem Sauerstoffanteil von > 15%.

Als wässrige Alkali-Hypochlorit-Lösungen wird bevorzugt eine NatriumhypochloritLösung verwendet. Besonders bevorzugt besitzt diese wässrige NatriumhypochloritLösung einen Gehalt an NaOCl von 1 bis 16 Gew.-%, insbesondere von 3 bis 12 Gew.-%.

Die Reaktion läuft vorzugsweise bei Temperaturen von 20 bis 200°C ab; in vielen Fällen hat es sich bewährt, bei Temperaturen von 30 bis 150°C, bevorzugt 40 bis 120°C, zu arbeiten.

Das erfindungsgemäße Verfahren kann drucklos, z.B. durch Durchleiten von Sauerstoff durch die Reaktionslösung, durchgeführt werden. Jedoch ist es für die Reaktionsgeschwindigkeit vorteilhaft, wenn ein Sauerstoffüberdruck angewandt wird. Das Verfahren kann bei Drücken bis zu 200 bar durchgeführt werden, wobei üblicherweise nur bis zu einem Druck von 60 bar gearbeitet wird und vorzugsweise im Bereich des Normaldrucks bis zu 20 bar.

Die Selektivität der Oxidationsreaktion und die Produktivität und Aktivität des Katalysators kann durch den Zusatz von Aminliganden teilweise verbessert werden.

Als Aminliganden können beispielsweise Pyridine, Bipyridine, Chinoline, Chinuclidinderivate, DABCO und Chinin- und Chinidinliganden verwendet werden.

Die eingesetzten Osmiumkatalysatoren sind ein oder mehrere verschiedene Osmiumverbindungen in den Oxidationsstufen +8 und +6. Es ist jedoch auch möglich, Osmiumpräkatalysatoren in niedrigeren Oxidationsstufen einzusetzen. Diese werden unter den Reaktionsbedingungen in die katalytisch aktiven Os(VIII)-Spezies umgewandelt. Als Osmium-Katalysatoren oder Katalysatorvorstufen können beispielsweise ein oder mehrere der im Folgenden genannten eingesetzt werden: OsO₄, K₂Os₂(OH)₄, Na₂Os₂(OH)₄, Os₃(CO)₁₂, OsCl₃, H₂OsCl₆. [CF₃SO₃Os(NH₃)₅](O₃SCF₃)₂, OsO₄ auf Vinylpyridin, BU^{t}NOsO₃.

Nach dem erfindungsgemäßen Verfahren wird der Osmiumkatalysator in katalytischen Mengen bezüglich des Alkohols (II) eingesetzt. Generell werden 0.02 bis 0.00001 Äquivalente bezogen auf Alkohol (II), bevorzugt 0.01 bis 0.0001 und besonders bevorzugt 0.008 bis 0.0005 Äquivalente verwendet.

Der Aminligand wird üblicherweise in einer Menge von 1:1 bis 100: 1 (bezogen auf Os) eingesetzt. Die Reaktion gelingt jedoch grundsätzlich auch ohne anwesenden Ligand.

Die Reaktionszeiten für das erfindungsgemäße Verfahren betragen in der Regel 8 bis 24 h und richten sich nach Substrat und Katalysatorkonzentration.

Prinzipiell führt man die Reaktion so durch, dass Osmiumkatalysator und gegebenenfalls der Ligand in die gepufferte wässrige Lösung unter Rühren gegeben wird.

Nach Zugabe des zu oxidierenden Alkohols wird das Oxidationsmittel (z.B. Hypochloritlösung) hinzugefügt. Bei Verwendung von Sauerstoff als Reoxidationsmittel wird das Reaktionsgefäß nach Spülen mit einem Niveaugefäß, gefüllt mit Sauerstoff verbunden, bzw. in einem Druckgefäß ein erhöhter Druck an Sauerstoff oder Luft aufgegeben. Die Reaktionslösung wird 12 bis 24 h gerührt, nach Zugabe von Natriumsulfit mit Essigester ausgeschüttelt und der Gehalt an Oxidationsprodukt und nicht umgesetztem Alkohol in der organischen Phase mittels GC bestimmt.

Der besondere Vorteil des erfindungsgemäßen Verfahrens ist die Verwendung von Sauerstoff oder sauerstoffenthaltenden Gasen als Reoxidationsmittel in Gegenwart nur sehr geringer Mengen an Osmiumkatalysator. Überraschenderweise können trotz der geringen Mengen an Osmium hohe Ausbeuten an Aldehyd bzw. Keton erreicht werden. Dies ist auf die hohe Selektivität und die große Reaktivität des Katalysatorsystems zurückzuführen.

Die Katalysatorproduktivität kann weiterhin erhöht werden, indem die einmal eingesetzte wässrige Katalysatorphase erneut mit Alkohol (II) versetzt wird. Dadurch werden die Katalysatorkosten für das erfindungsgemäße Verfahren minimiert, so dass technische Prozesse ökonomisch durchführbar sind.

Die besonderen Vorteile des neuen Prozesses bestehen in dem Preisvorteil des Oxidationsmittels, der Einfachheit der Durchführung und der großen Katalysatorproduktivität im Vergleich zu literaturbekannten Oxidationsreaktionen von Alkoholen. Die im erfindungsgemäßen Verfahren erreichten Katalysatorproduktivitäten (turnover numbers) von bis zu 20000 sind außergewöhnlich.

Die erfindungsgemäß hergestellten Carbonylverbindungen können unter anderem eingesetzt werden als Vorprodukte für Farbstoffe, Agrochemikalien, Kosmetika und Pharmazeutika.

Die nachstehenden Beispiele dienen zur Erläuterung des erfindungsmäßigen Verfahrens, ohne es darauf zu beschränken.

### Beispiel 1

In ein Schlenkgefäß werden 3.7 mg K₂OsO₄ . 2H₂O (0,01 mmol) eingewogen. Dazu werden unter Rühren mittels eines Magnetrührers 25 ml einer Pufferlösung vom pH 10,4, dargestellt aus 0,5 molarer K₂HPO₄-Lösung und 2 molarer NaOH, sowie 10 ml 2-Methyl-2-propanol gegeben, es bilden sich 2 Phasen. Das Gefäß wird in einem Wasserbad auf 50°C erwärmt und mit Sauerstoff gespült. Nach Zugabe von 206 µl Benzylalkohol (2 mmol) wird das Reaktionsgefäß mit einer Bürette, gefüllt mit Sauerstoff, verbunden, und die Reaktionslösung wird bei 50°C unter leichtem O₂₋Überdruck (ca. 50 cm Wassersäule) 18 Stunden gerührt.

Es wird wie im folgenden beschrieben aufgearbeitet:

Zu der Reaktionslösung werden ca. 1g Natriumbisulfit und 20 ml Essigester gegeben. Nach 10 minütigem Rühren wird die obere organische Phase abgetrennt. Mittels GC wird Benzaldehyd sowie nicht umgesetzter Alkohol bestimmt.
Ausbeute an Benzaldehyd 85% (Selektivität 85%).

### Beispiel 2

Es wird wie in Beispiel 1 angeführt verfahren, jedoch werden zu dem Osmiumsalz 0,03 mmol 1,4-Diazabicyclo[2,2,2]octan (DABCO) hinzugefügt.
Ausbeute an Benzaldehyd: 87% (Selektivität 87%).

### Beispiel 3

Es wird wie im Beispiel 2 angegeben verfahren, jedoch beträgt die Reaktionszeit 16 Stunden.
Ausbeute an Benzaldehyd: 93% (Selektivität 93%).

### Beispiel 4

Es wird wie in Beispiel 2 angeführt verfahren, jedoch wird Toluol anstelle von 2-Methyl-2-propanol eingesetzt, die Reaktionszeit betrug 24 h.
Ausbeute an Benzaldehyd: 89% (Selektivität 97%).

### Beispiel 5

Analog Beispiel 2 werden in einer Reaktionszeit von 22 Stunden 276 mg (2 mmol) 4-Methoxy-benzylalkohol umgesetzt.
Ausbeute an Anisaldehyd: 92% (Selektivität 92%)

### Beispiel 6

Wie in Beispiel 2 beschrieben, jedoch in einer Reaktionszeit von 12 Stunden, werden 242 µl (2 mmol) 1-Phenylethanol umgesetzt.
Ausbeute an Acetophenon: 95% (Selektivität 95%).

### Beispiel 7

Analog Beispiel 6 werden 276 µl (2 mmol) 1-(p-Tolyl)-ethanol umgesetzt.
Ausbeute an p-Methyl-acetophenon: 93% (Selektivität 93%).

### Beispiel 8

Es wird wie in Beispiel 2 angeführt verfahren, jedoch werden in 21 Stunden 282 µl (2 mmol) p-Methoxy-α-methyl-benzylalkohol umgesetzt.
Ausbeute an p-Methoxy-acetophenon: 92% (Selektivität 92%).

### Beispiel 9

Analog Beispiel 2 werden in einer Reaktionszeit von 19 h 2 mmol 3-(Hydroxymethyl)-thiophen umgesetzt.
Ausbeute an Thiophen-3-carboxaldehyd: 80% (Selektivität 80%).

### Beispiel 10

Es wird wie in Beispiel 2 angeführt verfahren, jedoch werden in 24 Stunden 2 mmol 1-(2-Furyl) ethanol umgesetzt.
Ausbeute an 2-Furyl-methyl-keton :85% (Selektivität 91%).

### Beispiel 11

Wie in Beispiel 2 angeführt, werden in 20 Stunden 2 mmol (-)-Borneol ((-):(+) = 96:4) umgesetzt.
Ausbeute an Campher (Summe der Enantiomeren):97% (Selektivität 97%).

### Beispiel 12

Analog Beispiel 2, jedoch bei einer Reaktionstemperatur von 80°C, werden 265 µl (2 mmol) Cyclooctanol 24 Stunden umgesetzt
Ausbeute an Cyclooctanon: 71% (Selektivität 93%).

### Beispiel 13

Analog Beispiel 12 werden 317 µl (2 mmol) 2-Octanol umgesetzt.
Ausbeute an 2-Octanon: 49% (Selektivität 93%).

### Beispiel 14

In einen Druckautoklaven, versehen mit einem Teflonrührer, werden 0,0004 mmol K₂OsO₄ x 2H₂O (0.2 ml einer frisch bereiteten Lösung von 7.4 mg K₂OsO₄ x 2H₂O in 10 ml Wasser), 0,0012 mmol DABCO (0.2 ml einer frisch bereiteten Lösung von 6.8 mg in 10 ml Wasser), 25 ml einer Pufferlösung vom pH 10,4, dargestellt aus 0,5 molarer K₂HPO₄-Lösung und 2 molarer NaOH, sowie 12 ml 2-Methyl-2-propanol gegeben. Nach Zugabe von 412 µl (4 mmol) Benzylalkohol wird ein Druck von 20 bar Luft aufgegeben und das Autoklavengefäß auf 80°C erwärmt.

Nach 24h Rühren bei 80°C lässt man abkühlen und entspannt. Man gibt ca. 200 mg Natriumbisulfit hinzu, schüttelt 2x mit je 20ml Essigester aus und bestimmt gaschromatografisch den Gehalt an Benzylalkohol und Benzaldehyd.
Ausbeute an Benzaldehyd: 90% (Selektivität 92%).

### Beispiel 15

Wie in Beispiel 14 angeführt, werden 4 mmol Benzylalkohol in Gegenwart von 0.0002 mmol K₂OsO₄. 2H₂O /0,0012 mmol DABCO 24 h bei 100°C und bei einem Druck von 40 bar Luft umgesetzt.
Ausbeute an Benzaldehyd: 86% (Selektivität 94%).

### Beispiel 16

Analog Beispiel 14 werden 4 mmol Phenylethanol umgesetzt.
Ausbeute an Acetophenon: 83% (Selektivität 91%).

### Beispiel 17

Analog Beispiel 15 werden 4 mmol Phenylethanol umgesetzt.
Ausbeute an Acetophenon: 75% (Selektivität 92%).

### Beispiel 18

In ein Schlenkgefäß werden 3.7 mg K₂OsO₄ . 2H₂O (0,01 mmol) gegeben. Dazu werden 25 ml einer Pufferlösung vom pH 10,4, dargestellt aus 0,5 molarer K₂HPO₄₋Lösung und 2 molarer NaOH, 4 ml einer 3 %igen Natriumhypochloritlösung sowie 10 ml 2-Methyl-2-propanol gegeben. Nach Zugabe von 265 µl (2 mmol) Cyclooctanol wird die Mischung 24h in einem Flüssigkeitsbad bei 70°C gerührt. Zu der Reaktionslösung 20 ml Essigester gegeben. Nach 10 minütigem Rühren wird die obere organische Phase abgetrennt. Mittels GC wird Cyclooctanon sowie nicht umgesetzter Alkohol bestimmt.
Ausbeute an Cyclooctanon: 56% (Selektivität 80%).

### Beispiel 19

Wie in Beispiel 18 angeführt, werden 2mmol 1-Phenylethanol unter Einsatz von 0,004 mmol K₂OsO₄ . 2H₂O (2 ml einer frisch bereiteten Lösung von 7.4 mg K₂OsO₄. 2 H₂O in 10 ml Wasser) und 10ml Hypochloritlösung 24h bei 50°C umgesetzt.
Ausbeute an Acetophenon: 74% (Selektivität 80%).

### Beispiel 20

Analog Beispiel 19 werden 2 mmol Benzylalkohol 24h bei 25°C umgesetzt.
Ausbeute an Benzaldehyd: 95% (Selektivität 97%).

### Beispiel 21

Wie in Beispiel 18 beschrieben, werden 2 mmol Benzylalkohol unter Einsatz von 0,001 mmol K₂OsO₄ . 2H₂O (0.5 ml einer frisch bereiteten Lösung von 7.4 mg K₂OsO₄ . 2H₂O in 10 ml Wasser) und 10ml Hypochloritlösung 24h bei 50°C umgesetzt.
Ausbeute an Benzaldehyd: 88% (Selektivität 90%).

## Patentansprüche

1. Verfahren zur Herstellung von Carbonylverbindungen der Formel (I), 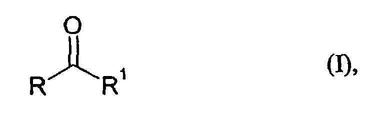 worin
R und R¹ unabhängig voneinander für Wasserstoff oder für einen jeweils gegebenenfalls substituierten Alkyl-, Cycloalkyl-, Aryl- oder Heteroarylrest stehen, wobei Cycloalkyl für einen gegebenenfalls 1- bis 8-fäch, gleich oder verschieden substituierten cyclischen Kohlenwasserstoffrest mit 5 bis 18 C-Atomen steht, wobei ein oder mehrere CH₂-Gruppen dieses Cycloalkylrestes durch Heteroatome ersetzt sein können,
oder
R und R¹ gemeinsam mit dem C-Atom, an welches sie gebunden sind, für eine Cycloalkylrest stehen, wobei Cycloalkyl für einen gegebenenfalls 1- bis 8-fach, gleich oder verschieden substituierten cyclischen Kohlenwasserstoffrest mit 5 bis 18 C-Atomen steht, wobei ein oder mehrere CH₂-Gruppen dieses Cycloalkylrestes durch Heteroatome ersetzt sein können,
durch Umsetzung von Alkoholen der allgemeinen Formel (II), 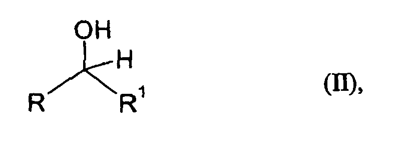 worin R und R¹ die oben genannte Bedeutung besitzen,
mit einem Oxidationsmittel in Gegenwart einer katalytischen Menge eines Osmiumkatalysators in Wasser oder einem Wasser enthaltenden Lösemittelgemisch bei einem pH-Wert von 7 bis 14, wobei als Oxidationsmittel molekularer Sauerstoff oder eine Gasmischung, die molekularen Sauerstoff enthält, oder eine Alkali- oder Erdalkali-Hypochlorit-Lösung verwendet wird, als Osmiumkatalysator ein oder mehrere verschiedene Osmiumverbindungen in den Oxidationsstufen +8 und +6 oder Osmiumpräkatalysatoren in niedrigeren Oxidationsstufen eingesetzt werden, wobei die Osmiumpräkatalysatoren in niedrigeren Oxidationsstufen unter den Reaktionsbedingungen in katalytisch aktive Os(VIII)-Spezies umgewandelt werden, und als Wasser enthaltendes Lösemittelgemisch eine Mischung aus Wasser und mindestens einem organischen Lösemittel aus der Reihe der tertiären Alkohole, Ester und aliphatischen Ether verwendet wird.

2. Verfahren gemäß Anspruch 1, **dadurch gekennzeichnet, dass** Alkohole der Formel (II) eingesetzt werden, worin mindestens einer der Reste R oder R¹ für eine Aryl- oder Heteroarylgruppe steht.

3. Verfahren gemäß Ansprüchen 1 und 2, **dadurch gekennzeichnet, dass** Alkohole der Formel (II) eingesetzt werden, worin
R und R¹ unabhängig voneinander für Wasserstoff, für jeweils geradkettiges oder verzweigtes, gegebenenfalls 1-bis 3-fach gleich oder verschieden substituiertes C₁-C₈-Alkyl, für gegebenenfalls 1-bis 3-fach gleich oder verschieden substituiertes C₅-C₁₀-Cyaoakyl, für gegebenenfalls substituiertes Phenyl oder Naphthyl oder für gegebenenfalls 1-bis 3-fach, gleich oder verschieden substituiertes C₅-C₇-Heteroaryl mit 1 oder 2 Heteroatomen aus der Reihe N,O,S stehen, oder
R und R¹ gemeinsam mit dem C-Atom, an welches sie gebunden sind, für gegebenenfalls 1-bis 3-fach gleich oder verschieden substituierten C₅-C₁₀₋Cycloalkyl stehen.

4. Verfahren nach mindestens einem der Ansprüche 1 bis 3, **dadurch gekennzeichnet, dass** als Oxidationsmittel Sauerstoff oder eine Gasmischung, die mindestens 15 Volumenprozent Sauerstoff enthält, verwendet wird.

5. Verfahren gemäß mindestens einem der Ansprüche 1 bis 4, **dadurch gekennzeichnet, dass** als Katalysator und/oder Präkatalysator mindestens eine Osmiumverbindungen aus der Gruppe OsO₄, K₂Os₂(OH)₄, Na₂Os₂(OH)₄, Os₃(CO)₁₂, O_{S}Cl₃, H₂OsCl₆, [CF₃SO₃Os(NH₃)₅](O₃SCF₃)₂, OsO₄ auf Vinylpyridin und Bu^{t}NOsO₃ eingesetzt wird.

6. Verfahren gemäß mindestens einem der Ansprüche 1 bis 5, **dadurch gekennzeichnet, dass** die Menge an eingesetztem Osmiumkatalysator 0.02 bis 0.00001 Äquivalente bezogen auf Alkohol der Formel (II) beträgt.

7. Verfahren nach mindestens einem der Ansprüche 1 bis 6, **dadurch gekennzeichnet, dass** die Reaktion bei einer Temperatur von 20 bis 200°C durchgeführt wird.

8. Verfahren gemäß mindestens einem der Ansprüche 1 bis 7, **dadurch gekennzeichnet, dass** die Reaktion bei einem Druck von bis zu 200 bar durchgeführt wird.

9. Verfahren gemäß mindestens einem der Ansprüche 1 bis 8, **dadurch gekennzeichnet, dass** mindestens ein Aminligand als Co-Katalysator zugesetzt wird.

## Claims

1. Process for the preparation of carbonyl compounds of the formula (I) 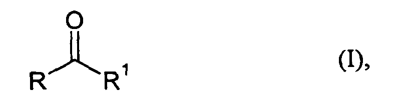 where
R and R¹ are independently hydrogen or an alkyl, cycloalkyl, aryl or heteroaryl radical, each of which is optionally substituted, where cycloalkyl represents a cyclic hydrocarbon radical having from 5 to 18 carbon atoms, which is optionally substituted by from 1 to 8 identical or different substituents, and one or more CH₂ groups of this cycloalkyl radical can be replaced by heteroatoms,
or
R and R¹ combine with the carbon atom to which they are bonded to form a cycloalkyl radical, where cycloalkyl represents a cyclic hydrocarbon radical having from 5 to 18 carbon atoms, which is optionally substituted by from 1 to 8 identical or different substituents, and one or more CH₂ groups of this cycloalkyl radical can be replaced by heteroatoms,
by reacting alcohols of the general formula (II) 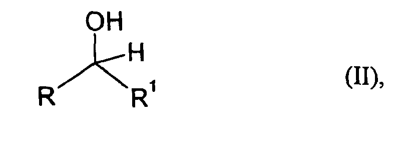 where R and R¹ are as defined above
with an oxidizing agent in the presence of a catalytic quantity of an osmium catalyst in water or in a solvent mixture containing water at a pH value of from 7 to 14, where the oxidizing agent used is molecular oxygen or a gas mixture which comprises molecular oxygen or an alkali metal or alkaline earth metal hypochlorite solution, the osmium catalyst used is one or more different osmium compounds in the +8 and +6 oxidation states or osmium precatalysts in lower oxidation states, where the osmium precatalysts in lower oxidation states are converted to catalytically active Os(VIII) species under the reaction conditions, and the water-containing solvent mixture used is a mixture of water and at least one organic solvent from the group of the tertiary alcohols, esters and aliphatic ethers.

2. Process according to Claim 1, **characterized in that** alcohols of the formula (II) are used where at least one of the radicals R and R¹ represents an aryl or heteroaryl group.

3. Process according to Claim 1 or 2, **characterized in that** alcohols of the formula (II) are used where
R and R¹ independently represent hydrogen, represent straight-chain or branched C₁-C₈-alkyl which is optionally substituted by from 1 to 3 identical or different substituents, represents C₅-C₁₀-cycloalkyl which is optionally substituted by from 1 to 3 identical or different substituents, represent optionally substituted phenyl or naphthyl, or represent C₅-C₇-heteroaryl which is optionally substituted by from 1 to 3 identical or different substituents and has 1 or 2 heteroatoms selected from the group consisting of N, O and S, or
R and R¹ together with the carbon atom to which they are bonded represent C₅-C₁₀-cycloalkyl which is optionally substituted by from 1 to 3 identical or different substituents.

4. Process according to any of Claims 1 to 3, **characterized in that** oxygen or a gas mixture comprising at least 15% by volume of oxygen is used as oxidizing agent.

5. Process according to any of Claims 1 to 4, **characterized in that** at least one osmium compound selected from the group consisting of OsO₄, K₂Os₂(OH)₄, Na₂Os₂(OH)₄, Os₃(CO)₁₂, OsCl₃, H₂OsCl₆, [CF₃SO₃Os(NH₃)₅](O₃SCF₃)₂, OsO₄ on vinylpyridine and Bu^{t}NOsO₃ is used as catalyst and/or precatalyst.

6. Process according to any of Claims 1 to 5, **characterized in that** the quantity of osmium catalyst used is in the range from 0.02 to 0.00001 equivalent based on the alcohol of formula (II).

7. Process according to any of Claims 1 to 6, **characterized in that** the reaction is carried out at a temperature of from 20 to 200°C.

8. Process according to any of Claims 1 to 7, **characterized in that** the reaction is carried out at a pressure of up to 200 bar.

9. Process according to any of Claims 1 to 8, **characterized in that** at least one amine ligand is added as cocatalyst.

## Revendications

1. Procédé de préparation de composés carbonyliques de formule (I) 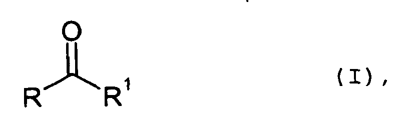 dans laquelle
R et R¹ représentent, indépendamment l'un de l'autre, un hydrogène ou un radical alkyle, cycloalkyle, aryle ou hétéroaryle, chacun éventuellement substitué, où le cycloalkyle représente un radical hydrocarboné cyclique éventuellement mono- à octat-substitué de façon identique ou différente, renfermant de 5 à 18 atomes de carbone, où un ou plusieurs groupes CH₂ de ce radical cycloalkyle peuvent être remplacés par des hétéroatomes,
ou
R et R¹ représentent, conjointement avec l'atome de carbone auquel ils sont liés, un radical cycloalkyle, où le cycloalkyle représente un radical hydrocarboné cyclique éventuellement mono- à octat-substitué de façon identique ou différente, renfermant de 5 à 18 atomes de carbone, où un ou plusieurs groupes CH₂ de ce radical cycloalkyle peuvent être remplacés par des hétéroatomes,
par réaction d'alcools de formule générale (II), 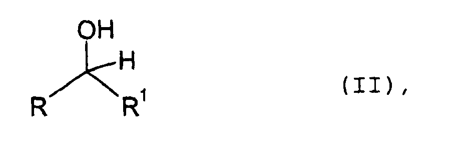 dans laquelle R et R¹ présentent la signification mentionnée ci-dessus,
avec un oxydant en présence d'une quantité catalytique d'un catalyseur à l'osnium dans de l'eau ou un mélange de solvants contenant de l'eau, à un pH de 7 à 14, où on utilise en tant qu'oxydant, de l'oxygène moléculaire ou un mélange gazeux contenant de l'oxygène moléculaire, ou une solution d'hypochlorite de métal alcalin ou de métal alcalino-terreux, on utilise, en tant que catalyseur à l'osnium, un ou plusieurs composés de l'osnium différents aux états d'oxydation +8 et +6 ou des pré-catalyseurs à l'osnium à des états d'oxydation inférieurs, où les pré-catalyseurs à l'osnium à des états d'oxydation inférieurs sont transformés en espèce Os(VIII) catalytiquement actives dans les conditions de réaction, et on utilise en tant que mélange de solvants contenant de l'eau, un mélange d'eau et d'au moins un solvant organique parmi le groupe des alcools tertiaires, des esters et des éthers aliphatiques.

2. Procédé selon la revendication 1, **caractérisé en ce que** l'on utilise des alcools de formule (II) dans laquelle au moins l'un des radicaux R ou R¹ représente un groupe aryle ou hétéroaryle.

3. Procédé selon les revendications 1 et 2, **caractérisé en ce que** l'on utilise des alcools de formule (II) dans laquelle
R et R¹ représentent, indépendamment l'un de l'autre, un hydrogène, un alkyle en C₁-C₈, dans chaque cas linéaire ou ramifié, éventuellement mono- à tri-substitué de façon identique ou différente, un cycloalkyle en C₅-C₁₀ éventuellement mono- à tri-substitué de façon identique ou différente, un phényle ou un naphtyle éventuellement substitué ou un hétéroaryle en C₅-C₇ éventuellement mono- à tri-substitué de façon identique ou différente, renfermant 1 ou 2 hétéroatomes parmi le groupe N, O et S, ou
R et R¹ représentent, conjointement avec l'atome de carbone auquel ils sont liés, un cycloalkyle en C₅₋C₁₀ éventuellement mono- à tri-substitué de façon identique ou différente.

4. Procédé selon au moins l'une des revendications 1 à 3, **caractérisé en ce que** l'on utilise en tant qu'oxydant, de l'oxygène ou un mélange gazeux contenant au moins 15 pour cent en volume d'oxygène.

5. Procédé selon au moins l'une des revendications 1 à 4, **caractérisé en ce que** l'on utilise en tant que catalyseur et/ou pré-catalyseur, au moins un composé de l'osnium parmi le groupe OsO₄, K₂Os₂(OH)₄, Na₂Os₂(OH)₄, Os₃(CO)₁₂, OsCl₃, H₂OsCl₆, [CF₃SO₃Os (NH₃)₅] (O₃SCF₃)₂, OsO₄ sur vinylpyridine et Bu^{t}NOsO₃.

6. Procédé selon au moins l'une des revendications 1 à 5, **caractérisé en ce que** la quantité de catalyseur à l'osnium utilisé est de 0,02 à 0,00001 équivalent, par rapport à l'alcool de formule (II).

7. Procédé selon au moins l'une des revendications 1 à 6, **caractérisé en ce que** la réaction est réalisée à une température de 20 à 200ºC.

8. Procédé selon au moins l'une des revendications 1 à 7, **caractérisé en ce que** la réaction est réalisée à une pression allant jusqu'à 200 bars.

9. Procédé selon au moins l'une des revendications 1 à 8, **caractérisé en ce que** l'on ajoute au moins un ligand aminé en tant que co-catalyseur.
